# EUROPEAN PATENT APPLICATION

(11) **EP 4 098 583 A1**
(43) Date of publication of application: **07.12.2022**
(21) Application number: 22176503.5
(22) Date of filing: 31.05.2022
(51) Int. Cl.: B65D 77/20, A61L 9/12

(54) **LID WITH A GRID AND A PEELABLE CLOSURE**

(30) Priority: 02.06.2021 ES 202131141 U
(71) Applicant: ZENIT Estudio de Diseño e Innovación S.L., 46010 Valencia (ES)
(72) Inventor: Blasco Feo, Vicente, 46010 Valencia (ES)
(74) Representative: Soler Lerma, Santiago

(57) **Abstract**

A lid (9) with a grid (6) and a peelable closure (5) wherein the closure comprises a film lid directly bonded to the grid without the need for third adhesive substances wherein the lid with the grid and the film form a single block in which all the points of contact between the film lid and the grid are welding points, said bond being obtained by contacting the film lid and the molten plastic inside the grid manufacturing mold.

## Description

The invention, as indicated by its name, refers to a lid with a grid, suitable for covering a container, especially those of volatile substances, allowing its contents to leak. The grid, made of plastic, is sealed during shipping and storage with a film sheet welded to the grid directly without the need for adhesives or glue, which has a welding point at each point of contact between the film sheet and the grid.

For this purpose, the film sheet is inserted and positioned in the grid manufacturing mold before the plastic is injected so that, when the molten plastic is injected at high temperatures to manufacture the grid, it is welded to the film sheet without melting it.

This avoids the use of adhesives and glues which, in addition to their environmental disadvantages, do not provide a satisfactory response since, in general, welding tends to be exclusively perimeter welding and, in addition, the vapors emanating from inside the container can usually degrade them, causing undesired leaks during transport and storage.

It belongs to the art sector of lids for volatile substances.

### BACKGROUND

There are diverse types of lids with grids on the market, especially in the sector of dosing devices for volatile substances, such as air fresheners or insecticides.

When a container is intended to contain a volatile substance that must be released once the product is in use, it is necessary to keep the volatile substance captured inside the container until it reaches its destination.

There are many ways of closing a container, the vast majority of which are not related to the type of closure that is the subject of this specification.

The volatile substances whose evaporation is of interest are sometimes embedded in small solid volumes such as flower petals or parts of plants, impregnated carriers, or scented pearls.

In these cases, it is convenient to provide the container with a grid, which normally forms part of the lid, so that, although volatile substances with scents, insecticides or repellents, among others, can easily pass into the atmosphere when the device is in use, the accidental overturning of the container does not cause the contents to fall out.

By «grid» we mean any perforated element arranged between the outside and the inside of the container in such a way that any communication between the inside and the outside of the container must necessarily pass through such perforations.

The challenge lies in preserving these volatile substances during shipping and storage of the product so, when it reaches the end user, its contents keep the origin packaging properties.

Among the solutions that have been found to this problem, it is worth mentioning a lid with a grid on its upper base sealed by a film glued with an easily removable adhesive or heat-sealed.

During shipping and handling operations for storage, either by the action of the vapors emanating from the container contents, or because the contents access the adhesive due to movement, it is easy for the adhesive to corrode and lose its airtightness.

The applicant herein developed a lid with a grid incorporating a closure that was attached, by geometry and pressure, to the grid area, covering it, all as described in patent EP3059183. This solution, perfectly valid from the point of view of efficiency, requires however the use of a quantity of plastic that the applicant wants to reduce.

To solve the above problems, a lid with a grid is proposed in which the grid is, during transport and storage operations, sealed by a sheet which is joined to the grid without the need for adhesives, which, in addition to being more economically and environmentally beneficial, proves to be more efficient since the joining is produced by a plurality of welding points.

### DESCRIPTION OF THE INVENTION

In order to solve the above problems, the lid comprises:
A support lid suitable for coupling to the neck of a container, wherein this lid comprises:
A perimeter wall defining an inner section. In a preferred embodiment, this perimeter wall is in the form of a circumference, although other executions are possible.

A grid arranged in the inner section of the perimeter wall in such a way that, the lid being attached to the neck of a container, the grid is suitable to allow the passage between the inside and the outside of the container. The term «grid» means herein the element arranged in the inner section of the lid regardless of whether it has perforations on its entire surface or only on part of it.

[1] A film sheet on the grid, adhered to it directly, without glues or adhesives, forming a single block with the lid. The film sheet completely covers the grid. This film sheet is fed into the grid manufacturing mold prior to injecting the plastic. The film sheet, at least on the side in contact with the grid, is made of a material, preferably plastic, different from that of the grid so that, when the film sheet comes into contact with the molten plastic that will form the grid, it does not melt the film sheet but allows the molten plastic to adhere to it.

Unlike other forms of sealing with film where adhesives are normally applied perimetrically, the bonding obtained between the film sheet and the grid in the claimed invention is perfect because welding points are created at all points of contact between the film sheet and the plastic of the grid and all this directly, i.e., without the need for glues or adhesives.

As welding points are generated at each point of contact between the plastic of the grid and the film sheet, the joint is consistent, preventing leakage of the contained product, since the film sheet completely covers the grid.

As mentioned above, the junction between the film sheet and the grid is very consistent, so that to facilitate peeling, understood as the removal of the film sheet to leave the grid uncovered, it is necessary to generate a grip point to pull it and remove it.

For this purpose, the film has a floating flange, which is separated from the lid, allowing it to be gripped and pulled to remove the film sheet.

In a preferred execution, this floating flange protrudes from the perimeter of the film sheet, although there may be an execution in which this floating flange does not protrude from the perimeter of the film sheet.

In one embodiment, to achieve the floating flange, the lid has a recess under the flange.

In a device such as the one advocated, the closure, in this case a film sheet, is not an add-on or a separate component that is glued once the elements are manufactured, but is incorporated during the manufacturing process of the grid as part of the assembly, which has several advantages:
It eliminates a step in the production line by not having to adhere the film closure to the grid once it has been molded.

It avoids the use of adhesives since the plastic that forms the grid, at the temperature at which it is injected into the mold, adheres to the film already placed inside the mold.

It benefits a much more homogeneous bond between the film and the grid since the molten plastic is evenly spread inside the mold.

It generates welding points at each contact point between the grid and the film sheet.

### BRIEF DESCRIPTION OF THE FIGURES

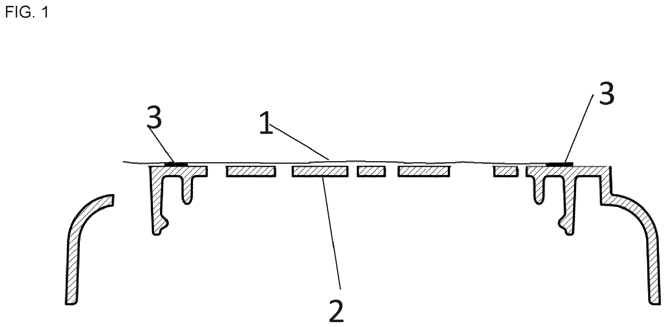

FIGURE 1 shows the state of the art in which the sealing film is glued to the grid (2) by means of adhesives (3), usually heat-sealing, arranged in its perimeter area. As can observed, the union does not take place between the grid cells due to the complexity that a heat-sealing matching with grid pattern would entail. This sealing, only perimetral, is a weak sealing because in addition to the fact that the bonding is only perimeter, the adhesives can degrade with the vapors emanating from the container.

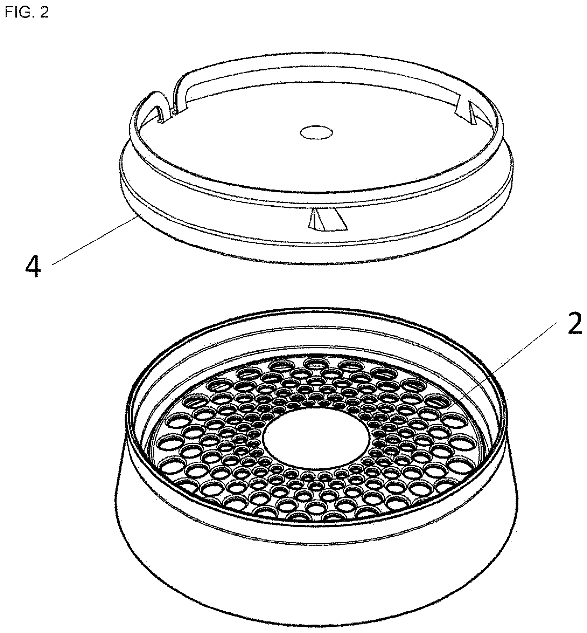

FIGURE 2 shows an exploded view of another solution existing in the prior art, specifically in patent EP3059183 where a rigid lid (4) is fixed on another grid (2) by geometry and pressure, thus avoiding the use of adhesives.
FIGURE 3 shows the claimed section of the invention wherein a film sheet (5) is directly adhered to a grid (6) being said film sheet welded to the grid at all those points where there is contact between the film sheet and the grid.

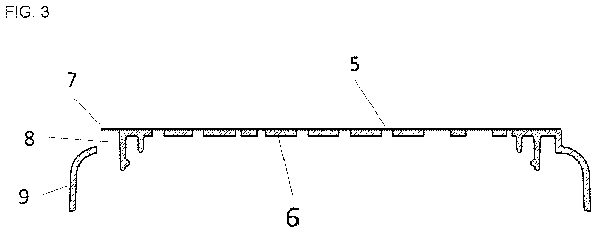

Figure 3 also shows one of the ends of the film sheet as a floating tab (7) arranged over a recess (8) of the lid (9) in such a way as to facilitate its gripping for the removal of the film sheet.

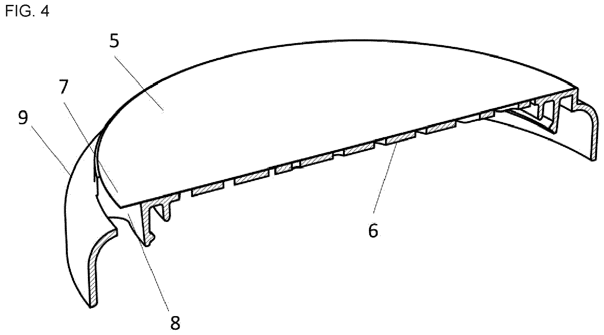

FIGURE 4 shows a section view of the lid (9) with the grid (6) sealed by the film sheet (5), showing at one end the flange (7) which flies over the recess (8) existing in the lid under the flange.

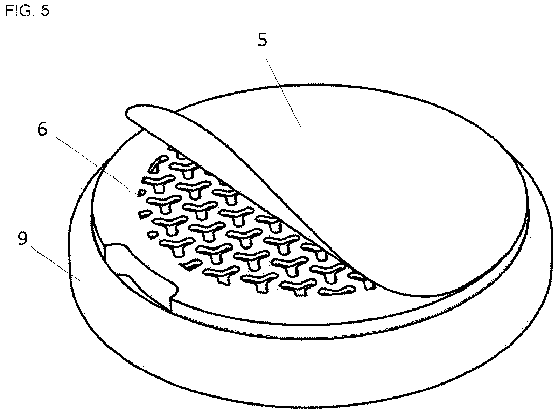

FIGURE 5 shows the lid (9) with a half-peeled film sheet (5) and the partial view of the grid (6).

### DESCRIPTION OF ONE EMBODIMENT OF THE INVENTION

An embodiment of the invention is set forth herein, which is neither unique nor limitative but explanatory.

The present invention relates to a lid (9) with a grid (6) incorporating a peelable closure.

The peelable closure comprises a film sheet (5) bonded directly to the grid, without the need for adhesives. The film sheet and the grid are fixed to each other at all points where they are in contact with each other, creating a very strong bond between the film sheet and the grid.

For this purpose, the film sheet and the grid are welded together during the manufacture of the grid, since the film sheet is previously positioned in the mold where the grid will be manufactured and thus, when the molten plastic is injected into the mold, the film sheet is welded to the plastic directly without the need for adhesives and at all points where the plastic contacts the film sheet.

The lid with grid and peelable closure as shown above comprises:
A lid (9) which supports the grid (6) and contributes to the connection with the container where the assembly is located.

A grid (6) arranged in the inner section of the lid support.

A film sheet (5) bonded directly to the grid (6) without the use of adhesives or glues.

The film sheet and the grid bond without the need for additional adhesives, since the union is produced by contacting, inside the mold, the film sheet previously inserted and the molten plastic that is fed into the mold for the manufacture of the grid.

This way of manufacturing creates a very solid bond because each of the contact points between the plastic and the film sheet is a welding point.

This bond is achieved by coinciding the film sheet and the molten plastic inside the grid manufacturing mold. For this purpose, the film sheet is fed into the mold and positioned before the molten plastic is injected, causing the molten plastic to weld to the film sheet and remain bonded to it once it has cooled.

The lid with grid and peelable closure thus produced behaves as a single block due to the multitude of welding points between the film sheet and the grid, since each point of contact between the two is a welding point.

The lid with grid and peelable closure thus produced behaves as a single block due to the multitude of welding points between the film sheet and the grid, since each point of contact between the two is a welding point.

For this purpose, an area of the film sheet, which we will call flange (7), is free, in the sense that it has no welding points.

This flange (7) is floating, i.e., it lacks welding points and is only connected to the grid through the rest of the film sheet, and, in a preferred execution, it overflies a gap (8) existing in the lid

## Claims

1. LID WITH A GRID AND A PEELABLE CLOSURE **characterized in that** it comprises a film sheet (5) directly bonded to the grid (6) forming a single block where all the points of contact between the film sheet and the grid are welding points, said union being obtained when the film sheet and the molten plastic come into contact inside the mold for manufacturing the grid.

2. LID WITH A GRID AND A PEELABLE CLOSURE according to claim 1, **characterized in that** the film sheet comprises a floating flange (7).

3. LID WITH A GRID AND A PEELABLE CLOSURE according to claim 2, **characterized in that** the flange (5) is arranged over a recess (8) of the lid (9).

4. LID WITH A GRID AND A PEELABLE CLOSURE according to claim 3, **characterized in that** the flange (5) protrudes from the regular perimeter of the film sheet.
